(19) Europäisches Patentamt
European Patent Office
Office européen des brevets

(11) EP 1 727 610 B1

## (12) EUROPÄISCHE PATENTSCHRIFT

(45) Veröffentlichungstag und Bekanntmachung des Hinweises auf die Patenterteilung:
**26.02.2014 Patentblatt 2014/09**

(21) Anmeldenummer: **05716152.3**

(22) Anmeldetag: **17.03.2005**

(51) Int Cl.:
***B01D 53/14*** *(2006.01)*  ***C07C 67/52*** *(2006.01)*
***C07C 69/82*** *(2006.01)*

(86) Internationale Anmeldenummer:
**PCT/EP2005/002847**

(87) Internationale Veröffentlichungsnummer:
**WO 2005/089905 (29.09.2005 Gazette 2005/39)**

(54) **KÜHLUNG UND REINIGUNG VON GASSTRÖMEN**

COOLING AND PURIFICATION OF GAS STREAMS

REFROIDISSEMENT ET PURIFICATION DE FLUX GAZEUX

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **19.03.2004 DE 102004013967**

(43) Veröffentlichungstag der Anmeldung:
**06.12.2006 Patentblatt 2006/49**

(73) Patentinhaber: **BASF SE**
**67056 Ludwigshafen (DE)**

(72) Erfinder:
• **LÖNING, Jan-Martin**
**67251 Freinsheim (DE)**
• **HECKMANN, Manfred**
**25050 Kuantan, Pahang (MY)**
• **HÖLEMANN, Karl**
**68199 Mannheim (DE)**
• **HEITZ, Thomas**
**67125 Dannstadt-Schauernheim (DE)**

(56) Entgegenhaltungen:
**DD-A1- 145 540      DD-A3- 160 829**
**US-A- 5 434 239      US-B1- 6 312 503**

EP 1 727 610 B1

**Beschreibung**

Die Erfindung betrifft ein verbessertes Verfahren zur Reinigung und Kühlung eines Dialkylesters A) einer aromatischen Carbonsäure enthaltenden Gasstromes.

Aromatische Dialkylester sind technisch bedeutende Ausgangsstoffe, beispielsweise zur Herstellung von Polyestern jeglicher Art.

Insbesondere Dimethylterephthalat (DMT) ist ein wichtiges Zwischenprodukt zur Herstellung verschiedener, technisch bedeutsamer Polyester wie z.B. Polyethylenterephthalat (PET) und Polybutylenterephthalat (PBT). DMT wird hierzu in geschmolzener Form mit den entsprechenden Alkoholen Ethylenglykol und 1,4-Butandiol katalytisch umgesetzt und die so gewonnenen monomeren Zwischenstufen anschließend durch Polykondensation in die Polyester überführt (Ullmann's Encyclopedia of Industrial Chemistry, 6th edition, 2000 Electronic Release, Wiley-VCH, Weinheim 2000).

Diese Dialkylester hydrolysieren bei Wasserkontakt sehr schnell, es bildet sich (Gleichgewichtsreaktion) die entsprechende Säure, die die Produktqualität des Polyesters negativ beeinflusst. Die Lagerung von DMT erfolgt in der Regel in geschmolzener Form bei Temperaturen von 165°C -170°C in inerter Atmosphäre (Stickstoff), damit eine Oxidation oder Hydrolyse des DMT verhindert wird und die Dosierung bei der Umesterung erleichtert wird. Aus der Tanklagerung werden bei kontinuierlicher Stickstoffnachspeisung somit DMT-beladene, heiße Gasströme freigesetzt.

Diese Gasströme entstehen im weiteren Polykondensationsprozess auch als Abgasströme aus den Polykondensationsreaktoren und Veresterungsstufen sowie Vorkondensationsstufen.

DMT neigt bei der Abkühlung zur Desublimation aus der Gasphase. Dieses kann zur Bildung von festen DMT-Partikein führen, die die Reinigung und Kühlung DMT-beladener Gasströme erheblich erschweren. Werden die festen DMT-Partikel nicht abgeschieden, können die zulässigen Emissionsgrenzwerte bezüglich DMT überschritten werden.

Aus der Literatur sind verschiedene Verfahren bekannt, die die Abscheidung von DMT aus Gasströmen durch Kontakt mit einer Flüssigkeit beschreiben.

In DD-A 160829 wird die Gaswäsche von DMT-haltigen Gasströmen aus einem PBT-Reaktor mit 1,4-Butandiol beschrieben. DMT wird hier durch das leichtsiedende Reaktionsnebenprodukt Methanol ausgestrippt, das destillativ abgetrennt wird. In einer Absorptionskolonne wird DMT durch 1,4-Butandiol als Schleppmittel mit einer Zulauftemperatur von 100 - 150°C vom Methanol-Trägergasstrom abgetrennt und in die Reaktionszone zurückgeführt. Eine gleichzeitige Gaskühlung wird nicht beschrieben.

Die Entfernung von DMT-Dämpfen und DMT-Partikel aus einer Tanklagerung von geschmolzenem DMT mit Hilfe von Wasser in einer Gleichstromapparatur wird in US 5749944 beschrieben. In einem einbautenlosen Apparat wird DMT durch Verdüsung von Wasser mit 10 - 32°C abgeschieden sowie der Trägergasstrom gleichzeitig abgekühlt. Als vorteilhaft wird hierbei der Verzicht auf niedrigsiedende organische Lösungsmittel (z.B. Methanol) beschrieben, so dass über den Reingasstrom keine zusätzlichen Emissionen des Waschmittels auftreten. Nachteilig erweist sich, das durch die starke Abkühlung am Apparateausgang ein nebelndes Gas-Flüssig-Gemisch mit festen DMT-Partikeln austritt, das eine weitere Trenneinheit zur Abscheidung der DMT-Partikel erforderlich macht. Durch den Kontakt mit Wasser besteht keine Rückfuhrmöglichkeit von DMT in den Syntheseprozess. Der Abwasserstrom muss der Entsorgung zugeführt werden. Zudem ist eine besondere Schutzeinrichtung erforderlich, die eine Rückströmung des Wassers über die Rohgasleitung in die DMT-Lagerung verhindert.

In DD-A 145540 wird die Abscheidung und Rückgewinnung von DMT bei der PET-Herstellung in einem mit Glykolverschluss versehenen DMT-Sublimatabscheider beansprucht. Die DMT beladenen Gasströme entstammen dem Zwischenlagern bzw. Aufschmelzen von DMT. Im Sublimatabscheider wird in der mittleren von 3 Kammern das DMT-haltige Gas mit Frischglykol mit einer Temperatur von 70-120°C im Gleichstrom geführt und durch Überdruck mit Inertgas über einen Glykolverschluss (20-160°C) hieraus in eine Austrittskammer des Gases gedrückt. Das im Glykol gelöste DMT kann somit in die PET-Synthese zurückgeführt werden. Eine zusätzliche Gaskühlung wird in diesem Verfahren nicht erwähnt.

Nachteilig erweist sich der Mitriß von DMT-Glykol Lösungen, die meist einen nachgeschalteten Abscheidebehälter erfordern.

Die Wäsche von DMT beladenen Gasströmen aus der DMT-Synthese in einem Gegenstromapparat mit Methanol wird in CS 134835 beschrieben. Das DMT kann nach einer Fest-Flüssig-Trennung in den Prozess zurückgeführt werden. Durch die Leicht-flüchtigkeit des Methanols ist ein zweiter Trennapparat notwendig, in dem durch eine Gaswäsche mit Wasser das Methanol aus dem Reingasstrom abgetrennt wird. Die vorliegenden Temperaturen werden nicht näher beschrieben. Eine DMT-Wäsche mit Methanol wird ebenfalls in der EP-A 0741124 beansprucht.

Zur Rückgewinnung des DMT aus Gasströmen können zudem Xylol (DE-A 2105017) sowie flüssiges DMT (US 3227743) eingesetzt werden.

Ein Verfahren zur gleichzeitigen Reinigung und Abkühlung von Gasströmen aus der PET-Synthese in einer zweistufigen Gegenstromwäsche mit Ethylenglykol wird in US 6312503 beschrieben. Der heiße Gasstrom (175°C) aus einem Polymerisationsreaktor zur PET-Herstellung enthält nicht näher spezifizierte Nebenprodukte sowie nicht umgesetzte Edukte, insbesondere Ethylenglykol, Acetaldehyd und Wasser.

**EP 1 727 610 B1**

Es wird dazu eine zweistufige Wäsche beansprucht, bei der im unteren Apparateabschnitt das Gas mit Abkühlraten kleiner 5,4°C/ft$^2$ (bezogen auf die Oberfläche der Einbauten) durch den direkten Kontakt mit einer Flüssigkeit abgekühlt wird. Mit der gleichen Flüssigkeit werden im oberen Apparateabschnitt dagegen bei geringeren Temperaturen die Fremdstoffe aus dem Inertgasstrom ausgewaschen.

Nachteilig an diesem Verfahren ist, dass nur eine Kühlung des Gasstromes erfolgt und im Apparat eine Nebelbildung auftritt wegen der sehr hohen Abkühlrate und da das untere Segment zum Quenchen (≙ Abkühlen) eingesetzt wird.

In DE-A 103 164 66.9 wird eine zweistufige Fahrweise vorgeschlagen, bei der die 1. Stufe oberhalb des Schmelzpunktes des Dialkylesters ausgeführt wird.

Aufgabe der vorliegenden Erfindung war es daher, ein verbessertes Verfahren zur Kühlung und Reinigung von eines Dialkylesters A) einer aromatischen Dicarbonsäure enthaltenden Gasstromes zur Verfügung zu stellen, welches dadurch gekennzeichnet ist, dass man in einer 1. Stufe den Gasstrom mit einer aliphatischen Dihydroxyverbindung B) bei einer Temperatur kleiner/gleich des Schmelzpunktes des Dialkylesters A) behandelt und in mindestens einer 2. Stufe den Gasstrom mit einer aliphatischen Dihydroxyverbindung B) oberhalb des Schmelzpunktes der Dihydroxyverbindung B) behandelt sowie dass in der ersten Stufe die Dihydroxyverbindung B) eine Temperatur kleiner/gleich 140 °C aufweist und in der 2. Stufe eine Temperatur von 20 bis 80 °C aufweist. Bevorzugte Ausführungsformen sind den Unteransprüchen zu entnehmen.

Überraschenderweise führt die erfindungsgemäße Verfahrensweise

- ökologisch und bezüglich Investitionskosten zu einer besseren Bilanz. Weiterhin wird

- der Verlust der aromatischen Dialkylester minimiert,

- der Energiebedarf für die untere Waschstufe minimiert und gleichzeitig der Waschmittelaustrag über die Gasphase von der unteren in die obere Waschstufe reduziert.

- der Gasstrom möglichst effizient gereinigt (Gehalt an Ester so gering wie möglich),

- der Ausgangsstoff Ester wieder in die Synthese zurückgeführt, d.h. die Raum-Zeit-Ausbeute ist erhöht,

- die Desublimation in der Vorrichtung wird verhindert und der Trägergasstrom gleichzeitig ohne Nebelbildung abgekühlt,

- die Diole weisen zudem eine hohe Löslichkeit für die Ester auf, so dass kein Feststoffausfall eintritt und ein Kreislaufbetrieb des Lösungsmittels ermöglicht wird.

Unter den Dialkylestern A) werden solche Verbindungen verstanden, welche aus einer aromatischen Dicarbonsäure mit aliphatischen Esterresten aufgebaut sind.

Als bevorzugte Dicarbonsäuren sind 2,6-Naphthalindicarbonsäure, Terephthalsäure und Isophthalsäure oder deren Mischungen zu nennen. Bis zu 30 mol-%, vorzugsweise nicht mehr als 10 mol-% der aromatischen Dicarbonsäuren können durch aliphatische oder cycloaliphatische Dicarbonsäuren wie Adipinsäure, Azelainsäure, Sebacinsäure, Dodecandisäuren und Cyclohexandicarbonsäuren ersetzt werden.

Bevorzugt werden Mischungen aus 5 bis 100 mol-% Isophthalsäure und 0 bis 95 mol-% Terephthalsäure, insbesondere Mischungen von etwa 80 % Terephthalsäure mit 20 % Isophthalsäure bis etwa äquivalente Mischungen dieser beiden Säuren verwendet.

Als ganz besonders bevorzugte Dicarbonsäure sei Terephthalsäure genannt.

Bevorzugte Alkylreste weisen 1 bis 4 C-Atome, insbesondere 1 bis 2 C-Atome auf. Bevorzugte Dialkylester sind solche, die sich von 2,6-Naphthalindicarbonsäure, Terephthalsäure und Isophthalsäure oder deren Mischungen ableiten, wobei die Dimethylester bevorzugt sind.

Insbesondere bevorzugt ist Dimethylterephthalat (DMT).

Als aliphatische Dihydroxyverbindung B) setzt man vorzugsweise Diole mit 2 bis 6 Kohlenstoffatomen ein, insbesondere 1,2-Ethandiol, 1,3-Propandiol, 1,2-Butandiol, 1,6-Hexandiol, 1,4-Hexandiol, 1,4-Butandiol, 1,4-Cyclohexandiol, 1,4-Cyclohexandimethylanol und Neopentylglykol oder deren Mischungen, wobei 1,4-Butandiol besonders bevorzugt ist.

Nachstehend sei das erfindungsgemäße Verfahren am Beispiel der Reinigung und Kühlung eines DMT-haltigen Gasstromes erläutert. Es sei jedoch betont, dass es für die Reinigung und Kühlung von Gasströmen verwendet werden kann, welche andere Dialkylester von aromatischen Dicarbonsäuren enthalten.

Bei der Tanklagerung wird DMT im allgemeinen in geschmolzenen Zustand bei Temperaturen von 140 bis

3

286°C, vorzugsweise 165 bis 170°C gehalten und mit trockenem Gasstrom, vorzugsweise Inertgasstrom, insbesondere Stickstoffstrom überlagert.

Durch vorzugsweise kontinuierliche Verdrängung des Inertgasstromes (wegen Tankbefüllung) entsteht ein DMT-beladener Gasstrom. Ein solcher Gasstrom entsteht auch als sog. Abgasstrom nach der Vor- bzw. Nachkondensation der Polyester, welcher in entsprechenderweise nach dem erfindungsgemäßen Verfahren gereinigt und gekühlt werden kann.

Hierzu wird der DMT-haltige Gasstrom in einer 1. Stufe mit den vorstehenden Dihydroxyverbindungen B) bei Temperaturen kleiner/gleich des Schmelzpunktes des Dialkylesters A) behandelt.

Für DMT beträgt der Schmelzpunkt 140°C, die Temperaturen des Gasstromes betragen in der Regel von 140 bis 286°C, vorzugsweise von 150 bis 170°C.

Als Vorrichtungen sind allgemein solche geeignet, welche sowohl im Gleichstrom als auch im Gegenstrom den Gasstrom in Kontakt bringen können. Das sind insbesondere Rieselfilmapparate, Füllkörper, Packungskolonnen, Apparate mit disperser flüssiger und kontinuierlicher Gasphase/Sprühapparate, Apparate mit Gas und kontinuierlicher Flüssigphase wie Blasensäulen oder Bodenkolonnen.

Zur Vergrößerung der Kontaktfläche zwischen den Phasen ist der entsprechende Apparat mit Einbauten wie Böden, Füllkörper, strukturierten Packungen sowie andere, dem Stand der Technik entsprechenden trennwirksamen Kolonneneinbauten ausgestattet. Der Apparat kann auch einbautenlos mit einer Verdüsung der Flüssigkeit ausgeführt werden. Die Komponente B) wird über herkömmliche Verteileinrichtungen oder Düsen in den Gasstrom eingebracht. Der Gas-Volumenstrom bei der Tanklagerung beträgt üblicherweise von 5 bis 75 m$^3$/h, vorzugsweise von 25 bis 50 m$^3$/h.

Der Anteil des DMT im Gasstrom ist durch den jeweiligen Sättigungsdampfdruck im entsprechenden Inertgasstrom begrenzt. Dieser beträgt in $N_2$ maximal 23 Gew.%, die Reinigung ist effektiv möglich bis zu einer Konzentration von 0,0001 Gew.-ppm DMT im $N_2$-Strom. Üblicherweise beträgt die Konzentration des DMT von 0,001 bis 16 Gew.-ppm, im $N_2$-Strom.

Bezogen auf den oben genannten temperaturabhängigen Sättigungsdampfdruck im entsprechenden Inertgasstrom z.B. $N_2$ von 23 Gew.-%, ist es vorteilhaft, das erfindungsgemäße Verfahren bei einem Sättigungsgrad [%] des Gasstromes von kleiner/gleich 50 %, vorzugsweise kleiner/gleich 40 % durchzuführen.

Der Sättigungsgrad des Gasstromes ist ein Maß für die Konzentration des DMT's in der Gasphase und ist definiert durch

$$\text{Sättigungsgrad [\%]} = \frac{\text{Partialdruck DMT}}{\text{Sättigungsdampfdruck DMT}} \cdot 100\,\%$$

$$\triangleq \text{ Maß für Konzentration DMT in der Gasphase}$$

(siehe Baehr, H.D., "Thermodynamik", 8. Auflage, Berlin u.a., Springer 1992; S. 212 ff).

Komponente B) wird gemäß des erfindungsgemäßen Verfahrens gleichzeitig oder anschließend z.B. über Verteilungsvorrichtungen z.B. Düsen oder bei Gegenstromfahrweise vorzugsweise am Ende der 1. Stufe zugegeben.

Die Temperatur in der 1. Stufe der Komponente B) wird durch den Schmelzpunkt der Komponente A) nach oben begrenzt. Für eine Fahrweise mit DMT als Ester beträgt dieser kleiner/gleich 140°C, vorzugsweise 140-50°C, und insbesondere 136-60°C, sowie ganz besonders bevorzugt 124-110°C.

Zur Verfahrensausführung können sowohl z.B. reines 1,4-Butandiol als auch mit DMT beladenes 1,4-Butandiol eingesetzt werden. Das 1,4-Butandiol kann sowohl im einfachen Durchgang sowie in Kreislaufbetrieb mit Rezirkulation mit dem Gas in Kontakt gebracht werden. Gas- und Flüssigphase können an jeder, dem oben beschriebenen Prinzip entsprechende Stelle im Apparat aufgegeben werden. Das Verfahrensprinzip kann gleichzeitig auf eine 3- und mehrstufige Betriebsweise ausgedehnt werden.

Der Druck in der 1. Stufe beträgt in der Regel von 1013 mbar (Umgebungsdruck, Normaldruck) bis 1113 mbar, vorzugsweise von 1013 bis 1083 mbar (für die Tanklagerung).

In der 1. Stufe wird erfindungsgemäß durch die Behandlung DMT aus der Gasphase in das flüssige Waschmittel (Dihydroxyverbindung) überführt.

Bei Austritt aus der 1. Stufe enthält der Gasstrom 0,01 bis 1000 Gew.-ppm DMT, vorzugsweise von 1 bis 50 Gew.-ppm DMT.

Der Flüssigkeitsstrom der Dihydroxyverbindung B) enthält bei Austritt aus der 1. Stufe 0,01 Gew.-ppm bis 59 Gew.-%, vorzugsweise von 0,1 Gew.-ppm bis 10 Gew.-% DMT.

Beim erfindungsgemäßen Verfahren wird in einer 2. Stufe der Gasstrom mit einer aliphatischen Dihydroxyverbindung B) gekühlt, wobei es wesentlich ist, dass in dieser Stufe oberhalb des Schmelzpunktes der Komponente B) gearbeitet wird. Die Temperatur beträgt entsprechend für 1,4-Butandiol von 20 bis 80°C und insbesondere von 50 bis 70°C, bei Ethylenglykol und Propandiol betragen die Schmelzpunkte -10°C bzw. - 32°C, so dass eine Arbeitsweise bei den o.g. Temperaturbereichen ebenso empfohlen wird.

Die hierfür geeigneten Vorrichtungen bzw. deren Einbauten entsprechen den Ausführungen bei Stufe 1.

Dies gilt auch für den Druck.

Die Temperaturparameter des erfindungsgemäßen Verfahrens sind so gestaltet, dass der tatsächliche DMT-Partialdruck (Gehalt in der Gasphase) den Partialdruck der beginnenden Desublimation nicht überschreitet.

Bei Austritt aus der zweiten Zone (welche auch in mehrere Zonen aufteilbar sein kann) weist der Gasstrom einen DMT-Gehalt von 0,001 bis 16 Gew.-ppm, vorzugsweise von 0,01 bis 1 Gew.-ppm auf.

Im folgenden sei eine besonders bevorzugte Ausführungsform (s. Abbildung) des erfindungsgemäßen Verfahrens näher erläutert:

In einem Tanklager (1) wird DMT in geschmolzenem Zustand gehalten und zur Vermeidung von Oxidation und Wasserkontakt mit trocknem Inertgas (2) (z.B. Stickstoff) überlagert. Durch kontinuierliche Verdrängung des Inertgases (z.B. bei Tankbefüllung) wird ein DMT-beladener Gasstrom (3) über eine beheizte Gasleitung sowie einen beheizten Gaseinlass (4) auf eine Absorptionskolonne (5) gegeben. Im unteren Kolonnenabschnitt (6) der mit trennwirksamen Einbauten gefüllt ist, wird dem Gasstrom über eine Verteileinrichtung (7) flüssiges 1,4-Butandiol mit einer Temperatur von 50°C < T < 139°C (je nach Sättigungsgrad) entgegengeführt. Hierdurch wird DMT aus der Gasphase in das flüssige Waschmittel überführt. Der Flüssigkeitsstrom (8) wird über einen Wärmetauscher (9) auf die Zulauftemperatur vorgeheizt. Der Strom kann sowohl dem Sumpfaustrag der Kolonne (10) als Teilstrom (11) unter Zumischung von reinem 1,4-Butandiol (12) entnommen werden oder als reines 1,4-Butandiol (12) zugeführt werden.

Der an DMT abgereicherte Gasstrom wird einem zweiten Kolonnenabschnitt (13) zugeführt, der mit trennwirksamen Einbauten gefüllt ist, und dort durch direkten Kontakt mit einem zweiten Waschmittelstrom (14) bei einer Temperatur von 20°C < T < 140°C abgekühlt wird. Der Waschmittelstrom wird über eine Verteileinrichtung (15) in die Kolonne gegeben und über einen Wärmetauscher (16) temperiert. Als Waschmittelstrom kann reines 1,4-Butandiol (12) sowie ein Rückfuhrstrom (17) aus dem Sumpfaustrag (10) der Absorptionskolonne verwendet werden. Der gereinigte Trägergasstrom (18) verlässt den Apparat über Kopf.

Durch die oben beschriebene Prozessführung wird die Desublimation von DMT im Apparat verhindert und der Gasstrom gleichzeitig ohne Nebelbildung abgekühlt. Ein feststofffreier Gasstrom wird abgegeben. Bei einer Ausführung in zwei getrennten Apparaten, ermöglicht die appartive Integration von DMT-Abtrennung und Gaskühlung in einer Absorptionskolonne günstigere Betriebs- und Investitionskosten.

Durch die Gaswäsche mit 1,4-Butandiol wird der Wertstoff DMT in einem prozesseigenen Lösungsmittel in das PBT-Verfahren zurückgeführt und die Gesamtausbeute bezogen auf DMT verbessert. Die Rückführung des DMT kann ohne zusätzliche Aufarbeitungsschritte direkt in den Veresterungsreaktor erfolgen, in dem DMT mit 1,4-Butandiol katalytisch verestert wird.

Im Vergleich zu den bereits bekannten Absorptionsverfahren mit niedrigsiedenden Lösungsmitteln (z.B. Methanol), wird durch die zweistufige Gaswäsche mit hochsiedendem 1,4-Butandiol der Lösungsmittelverlust über den Gasaustrag des Wäschers reduziert. 1,4-Butandiol weist zudem eine hohe Löslichkeit für DMT auf, so dass aus der Flüssigphase kein Feststoffausfall auftritt. Ein Kreislaufbetrieb des Lösungsmittels ist daher möglich. Die Raum-Zeit-Ausbeute ist daher beim anschließenden Polykondensationsverfahren zur Herstellung von Polyestern, insbesondere Polybutylenterephthalat (PBT), PET oder PTT signifikant erhöht.

Beispiel

DMT wird in einem Tanklager bei einer Temperatur von 170°C gelagert. Die Gasatmosphäre enthält 4,8 Gew.-% DMT und 95,2 Gew.-% Stickstoff. Bei der Tankentleerung wird typischerweise ein Gasstrom von 41 kg/h abgegeben. Der Gasstrom wird auf einem zweistufigen Wäscher mit einem Durchmesser von 200 mm gegeben und dort im unteren Apparateabschnitt mit 370 kg/h reinem 1,4-Butandiol mit einer Temperatur von 124 °C im Gegenstrom über trennwirksame Einbauten gewaschen. Im oberen Apparateabschnitt wird das Gas durch die Gegenstromführung mit 125 kg/h reinem 1,4-Buttandiol, das eine Zulauftemperatur von 60 °C besitzt, über trennwirksame Einbauten abgekühlt. Der auf 60,15 °C abgekühlte Reingasstrom wird mit einem Gehalt von kleiner 0,1 Gew.-% Butandiol und kleiner 0,2 Gew.-ppm DMT abgegeben. DMT-Bilanz: 99,98 % DMT-Rückgewinnung.

Im dargestellten Anwendungsbeispiel haben Gas- und Flüssigkeit beim Übergang vom unteren (heißes BD als Waschmittel) in den oberen Apparateabschnitt (kaltes BD) folgende Zusammensetzungen:

| Phase | Wert | |
|---|---|---|
| Gas | DMT | 24 Gew.-ppm |
| | 1,4-BD | 2,2 Gew.-% |
| | N2 | 97,8 Gew.-% |
| Flüssigkeit | DMT | 2,2 Gew.-ppm |
| | 1,4-BD | 99,99 Gew.-% |
| | N2 | 14 Gew.-% |

Im dargestellten Anwendungsbeispiel hat die Gasphase beim Austritt aus dem Apparat folgende Zusammensetzung:

| Phase | Wert | |
|---|---|---|
| Gas | DMT | 0,2 Gew.-ppm |
| | 1,4-BD | 0,07 Gew.-% |
| | N2 | 99,93 Gew.-% |

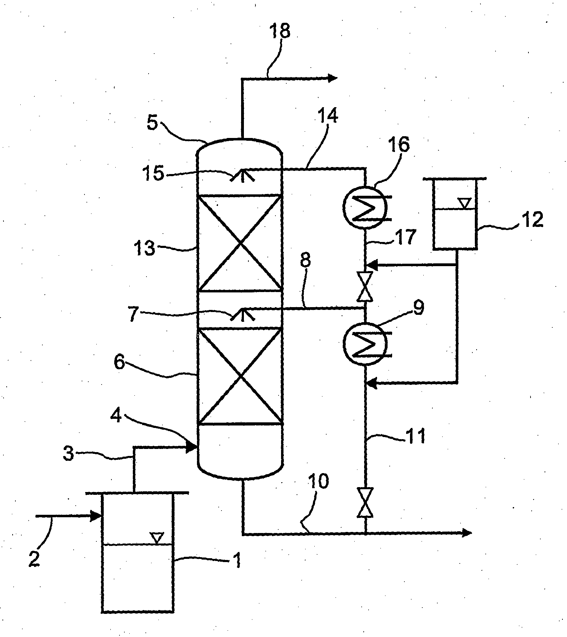

**Patentansprüche**

1.   Verfahren zur Reinigung und Kühlung eines Dialkylesters A) einer aromatischen Dicarbonsäure enthaltenden Gasstromes, **dadurch gekennzeichnet, dass** man in einer 1. Stufe den Gasstrom mit einer aliphatischen Dihydroxyverbindung B) bei einer Temperatur kleiner/gleich des Schmelzpunktes des Dialkylesters A) behandelt und in mindestens einer 2. Stufe den Gasstrom mit einer aliphatischen Dihydroxyverbindung B) oberhalb des Schmelzpunktes der Dihydroxyverbindung B) behandelt sowie dass in der ersten Stufe die Dihydroxyverbindung B) eine Temperatur kleiner/gleich 140°C aufweist und in der zweiten Stufe eine Temperatur von 20 bis 80°C aufweist.

2.   Verfahren nach Anspruch 1, **dadurch gekennzeichnet, dass** man als Dialkylester A) Ester der Terephthalsäure, Isophthalsäure, 2,6-Naphthalindicarbonsäure oder deren Mischungen einsetzt.

3.   Verfahren nach den Ansprüchen 1 oder 2, **dadurch gekennzeichnet, dass** man Dialkylester A) einsetzt, welche Alkylreste mit 1 bis 4 C-Atomen aufweisen.

4.   Verfahren nach den Ansprüchen 1 bis 3, **dadurch gekennzeichnet, dass** man als Gasstrom einen beladenen Inertgasstrom reinigt und kühlt.

5.   Verfahren nach den Ansprüchen 1 bis 4, **dadurch gekennzeichnet, dass** man als Dihydroxyverbindung B) Diole mit 2 bis 6 C-Atomen einsetzt.

6.   Verfahren nach den Ansprüchen 1 bis 5, **dadurch gekennzeichnet, dass** man als Dihydroxyverbindung B) 1,4-Butandiol einsetzt.

7.   Verfahren nach den Ansprüchen 1 bis 6, **dadurch gekennzeichnet, dass** man als Dialkylester A) Dimethylterephthalat einsetzt.

8.   Verfahren nach den Ansprüchen 1 bis 7, **dadurch gekennzeichnet, dass** der Sättigungsgrad [%] des Gasstroms bezüglich des Dialkylesters kleiner/gleich 50 % beträgt.

9.   Verfahren nach den Ansprüchen 1 bis 8, **dadurch gekennzeichnet, dass** der Gasstrom nach Reinigung und Kühlung weniger als 20 Gew.-ppm des aromatischen Dialkylesters A) enthält.

**Claims**

1.   A process for purifying and cooling a gas stream comprising a dialkyl ester A) of an aromatic dicarboxylic acid, which comprises treating the gas stream with an aliphatic dihydroxy compound B) at a temperature less than/equal to the melting point of the dialkyl ester A) in a 1st stage and treating the gas stream with an aliphatic dihydroxy compound B) at above the melting point of the dihydroxy compound B) in at least one second stage, wherein the dihydroxy compound B) has a temperature less than/equal to 140°C in the first stage and has a temperature of from 20 to 80°C in the second stage.

2. The process according to claim 1, wherein the dialkyl ester A) is an ester of terephthalic acid, isophthalic acid, 2,6-naphthalenedicarboxylic acid or a mixture thereof.

3. The process according to claim 1 or 2, wherein the dialkyl ester A) has alkyl radicals having from 1 to 4 carbon atoms.

4. The process according to any of claims 1 to 3, wherein the gas stream which is purified and cooled is a laden inert gas stream.

5. The process according to any of claims 1 to 4, wherein the dihydroxy compound B) used is a diol having from 2 to 6 carbon atoms.

6. The process according to any of claims 1 to 5, wherein the dihydroxy compound B) used is 1,4-butanediol.

7. The process according to any of claims 1 to 6, wherein the dialkyl ester A) is dimethyl terephthalate.

8. The process according to any of claims 1 to 7, wherein the degree of saturation[%] of the gas stream with respect to the dialkyl ester is less than/equal to 50%.

9. The process according to any of claims 1 to 8, wherein the gas stream contains less than 20 ppm by weight of the aromatic dialkyl ester A) after purification and cooling.

**Revendications**

1. Procédé pour la purification et le refroidissement d'un flux gazeux contenant un ester dialkylique A) d'un acide dicarboxylique aromatique, **caractérisé en ce que**, dans une première étape, on traite le flux gazeux avec un composé aliphatique à fonctionnalité dihydroxy B) à une température inférieure ou égale au point de fusion de l'ester dialkylique A) et, dans au moins une deuxième étape, on traite le flux gazeux avec un composé aliphatique à fonctionnalité dihydroxy B) au-dessus du point de fusion du composé à fonctionnalité dihydroxy B) et **en ce que** dans la première étape, le composé à fonctionnalité dihydroxy B) présente une température inférieure ou égale à 140°C et, dans la deuxième étape, une température de 20 à 80°C.

2. Procédé selon la revendication 1, **caractérisé en ce qu'**on utilise, comme esters dialkyliques A), des esters de l'acide téréphtalique, de l'acide isophtalique, de l'acide 2,6-naphtalènedicarboxylique ou leurs mélanges.

3. Procédé selon les revendications 1 ou 2, **caractérisé en ce qu'**on utilise des esters dialkyliques A) qui présentent des radicaux alkyle de 1 à 4 atomes de carbone.

4. Procédé selon les revendications 1 à 3, **caractérisé en ce qu'**on purifie et refroidit, comme flux gazeux, un flux de gaz inerte chargé.

5. Procédé selon les revendications 1 à 4, **caractérisé en ce qu'**on utilise, comme composé à fonctionnalité dihydroxy B), des diols comprenant 2 à 6 atomes de carbone.

6. Procédé selon les revendications 1 à 5, **caractérisé en ce qu'**on utilise, comme composé à fonctionnalité dihydroxy B), du 1,4-butanediol.

7. Procédé selon les revendications 1 à 6, **caractérisé en ce qu'**on utilise, comme ester dialkylique A), du téréphtalate de diméthyle.

8. Procédé selon les revendications 1 à 7, **caractérisé en ce que** le degré de saturation [%] du flux gazeux, en ce qui concerne l'ester dialkylique, est inférieur ou égal à 50%.

9. Procédé selon les revendications 1 à 8, **caractérisé en ce que** le flux gazeux contient, après la purification et le refroidissement, moins de 20 ppm en poids de l'ester dialkylique aromatique A).

EP 1 727 610 B1

9

**IN DER BESCHREIBUNG AUFGEFÜHRTE DOKUMENTE**

**In der Beschreibung aufgeführte Patentdokumente**

- DD 160829 A
- US 5749944 A
- DD 145540 A
- US 134835 A
- EP 0741124 A
- DE 2105017 A
- US 3227743 A
- US 6312503 B
- DE 10316466 A

**In der Beschreibung aufgeführte Nicht-Patentliteratur**

- Ullmann's Encyclopedia of Industrial Chemistry. Wiley-VCH, 2000